Europäisches Patentamt

European Patent Office

Office européen des brevets

(19)

(11) Publication number: **0 042 412**
**B1**

# EUROPEAN PATENT SPECIFICATION

(12)

(45) Date of publication of the patent specification:
18.04.84

(51) Int. Cl.³: **C 07 C 41/01**

(21) Application number: **81900246.0**

(22) Date of filing: **10.12.80**

(86) International application number:
**PCT/US 80/01675**

(87) International publication number:
**WO 81/01846 (09.07.81 Gazette 81/16)**

(54) **METHOD OF SYNTHESIZING FLUOROMETHYLHEXAFLUOROISOPROPYL ETHER.**

(30) Priority: **26.12.79 US 107117**

(43) Date of publication of application:
**30.12.81 Bulletin 81/52**

(45) Publication of the grant of the patent:
**18.04.84 Bulletin 84/16**

(84) Designated Contracting States:
**CH DE FR GB LI**

(56) References cited:
**US - A - 2 992 276**
**US - A - 3 683 092**
**US - A - 3 689 571**

(73) Proprietor: **BAXTER TRAVENOL LABORATORIES, INC.,**
**One Baxter Parkway, Deerfield, IL 60015 (US)**

(72) Inventor: **COON, Clifford L., 37063 Shasta St., Fremont,**
**CA 94536 (US)**
Inventor: **SIMON, Robert L., 129 Beverly Drive, San**
**Carlos, CA 94070 (US)**

(74) Representative: **MacGregor, Gordon et al, ERIC POTTER**
**& CLARKSON 14 Oxford Street, Nottingham, NG1 5BP**
**(GB)**

Method of Synthesizing Fluoromethylhexafluoroisopropyl Ether

*Related Copending Application*

This is a continuation-in-part of U.S. Patent Application Serial No. 107117, filed December 26, 1979.

*Background of the Invention*

Fluoromethyl-1,1,1,3,3,3-hexafluoroisopropyl ether, as described in U.S. Patent Nos. 3683092 and 3689571, is a promising new anesthetic for human use which is essentially non-inflammable and appears to have few or no undesirable side effects when administered to humans.

In the abandoned Patent Application Serial No. 771365, filed October 28, 1978, from which the above two patents claim priority, several techniques are suggested which may be used for making ethers having halogen groups in both of the organic ether substituents, including fluoromethylhexafluoroisopropyl ether. It is suggested there that the corresponding alcohol may be reacted with formaldehyde and hydrogen fluoride to form the fluoromethyl ether. However, yields of this reaction, generally described in the abandoned patent application cited above, are not of a desired commercial scale, so other, more cumbersome, multiple step synthesis route were initially preferred. Weynmayr U.S. Patent No. 2992276 also teaches the use of paraformaldehyde and hydrogen fluoride as a reagent for synthesizing a fluoromethylether and an alcohol from tetrafluoroethylene.

In accordance with this invention, a simplified, high-yield synthesis technique for fluoromethylhexafluoroisopropyl ether is disclosed, capable of producing yields of the desired ether product of the order of 90%, with recycling of unused reactants through the reaction mixture for optimization of synthesis. Particularly fluoromethyl-1,1,1,3,3,3-hexafluoroisopropyl ether may be made this way as a clinical anesthetic on a large scale basis.

*Summary of the Invention*

In accordance with this invention, a method of synthesizing fluoromethylhexafluoroisopropyl ether is provided, in which formaldehyde, hydrogen fluoride and hexafluoroisopropyl alcohol are reacted together, characterized by the inclusion of a Lewis acid or a protonating, dehydrating and fluoride ion-generating agent in the reaction mixture.

*Brief Description of the Drawing*

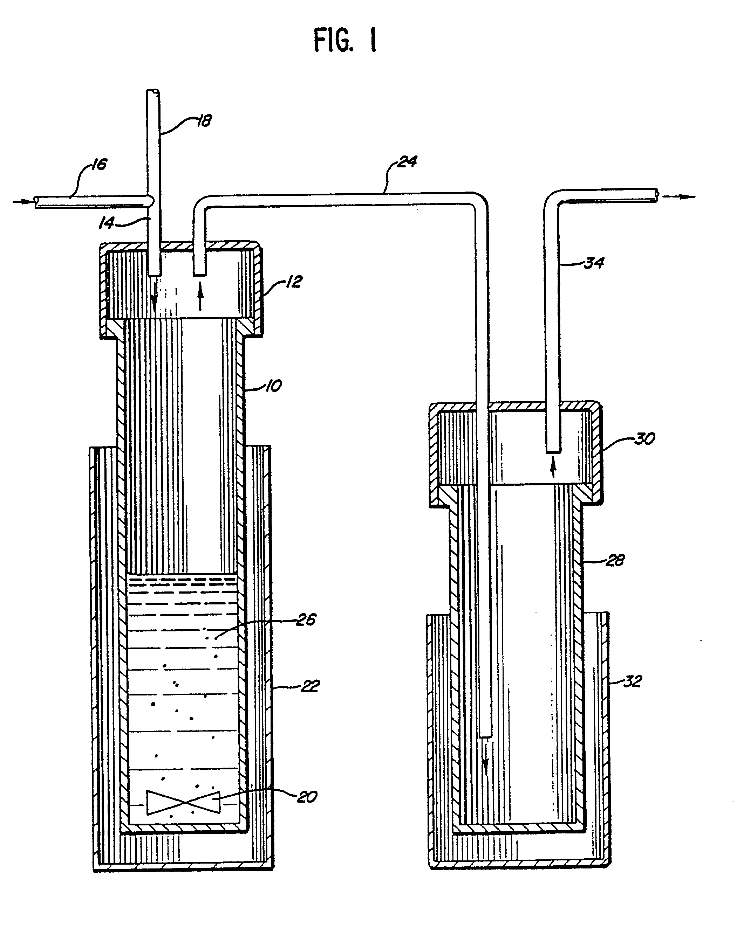

Fig. 1 is a schematic view of the reaction apparatus used for performing the reaction described above, with further details of the reaction technique added.

Reffering to Fig. 1, reaction vessel 10, made of Kel-F fluorinated plastic and sealed with closure 12, defines an inlet line 14 which has a branch connection. One of the connection 16 is connected to a source of pressurized inert, e.g., nitrogen gas, and the other connection 18 is connected to a source of hexafluoroisopropyl alcohol. The reaction vessel 10 is equipped with a magnetic stirring bar 20 and positioned within an oil bath 22 for control of the temperature of the reaction mixture at, preferably, about 65°C.

Tubular outlet line 24 communicates with container 10 and carries vapors generated by the reaction mixture 26, to a collector container 28 made of Kel-F fluorinated plastic. Container 28 also defines closure 30 with line 34 sealingly passing through it. Container 28 is also placed in a cooling bath 32 to assist in condensation of the vapors in container 32.

Vent line 34 communicates with the exterior. Accordingly, nitrogen gas may be constantly used to provide a low velocity gas sweep through the reaction system, while the alcohol reactant is added dropwise through inlet line 18. The vapors which are generated leave reaction chamber 10 through line 24 and are condensed in container 28. The sweeping nitrogen gas then continues to pass outwardly through vent 34, while the products and byproducts of the reaction are collected in the collector container 28.

*Detailed Description of the Preferred Embodiments*

While the order of addition of reactants is not critical, the synthesis is preferably conducted by slowly adding the hexafluoroisopropyl alcohol to a preformed mixture of formaldehyde, hydrogen fluoride and the dehydrating, protonating and fluoride ion-generating agent.

The identity of the selected agent is not critical so long as the protonating, dehydrating and fluoride ion-generating functions are accomplished to at least some degree in the reaction mixture. As a principal feature, the agent must be capable of sequestering water generated during the reaction. The effectiveness of a proposed agent for such purpose may be readily determined in a pilot synthesis by simply assaying for the presence of free water in the reaction mixture.

The agent should also be a protonating agent in the reaction of hexafluoroisopropyl alcohol with formaldehyde and hydrogen fluoride and should generate fluoride ions in the same reaction. Agents which exhibit these characteristics enhance yields to greater than about 40% fluoromethylhexafluoroisopropyl ether based on the weight of the alcohol starting material.

The preferred agents are Brönsted acids having a relative acid strength in excess of about 15 for the first proton, in particular sulfuric acid (relative strength 39), fluorosulfonic acid or trifluoromethanesulfonic acid (both 427). Mixtures may also be employed; sulfuric acid or a mixture of fluorosulfonic acid and sulfuric acid have produced the best yields to date.

A Lewis acid may be used instead of a protonating agent such as a Brönsted acid. The Lewis acid may be titanium tetrachloride, aluminium trichloride, aluminium trifluoride or antimony pentafluoride.

The term formaldehyde as used herein is intended to include formaldehyde polymers such as paraformaldehyde, which is preferred.

The reaction temperature is not critical, but yields are considerably improved above about 50°C. Preferably, the temperature of the reaction mixture is maintained at about 57 to 70°C, with the hexafluoroisopropyl alcohol being added on a continuous, gradual basis. This permits the distillation of the ether product concurrent with its synthesis, thereby reducing degradation of the product when exposed to the harsh conditions or the reaction mixture.

Both the formaldehyde and the hydrogen fluoride are present optimally in a stoichiometric excess compared to the total amount of alcohol to be used. Preferably, at least about from 10 to 100% molar excess of formaldehyde and at least about from 400 to 1,000% excess of hydrogen fluoride are present in the reaction mixture.

It is preferable that at least half again as much agent as formaldehyde by weight be present. Preferably, about from 50 to 200% greater weight of a Brönsted acid such as sulfuric acid will be employed.

The fluoromethylhexafluoroisopropyl ether is recovered from the reaction mixture in any known fashion, preferably by condensing vapors generated during the reaction. Recovery of product is aided by employing a reaction temperature in excess of about 57°C, the boiling point of fluoromethylhexafluoroisopropyl ether. A major proportion of the condensate is the starting alcohol and the ether product. It is preferably neutralized where a volatile acid had been used as the agent. The condensate can then be redistilled to improve the ether purity. A fraction distilling at about from 58 to 95°C principally contains the alcohol starting material, while distillation at a temperature below about 58°C will yield the ether. The alcohol-containing fraction may be recycled to the reaction mixture. Thus, the process may be conducted continuously if the recycled fraction, formaldehyde, hydrogen fluoride and supplementary starting alcohol are added as required.

It is contemplated that other ingredients such as solvents, catalysts, diluents, and other materials may also be present in the reaction mixture if desired, as long as the added extraneous materials do not materially change the nature of the reaction described above, but are added to promote the reaction, suppress side reactions, or improve the purification step of the synthesis.

The following examples are presented for illustrative purposes only, and are not intended to limit the scope of the invention, which is as defined in the claims below. All analyses were conducted by gas chromatography. All percentages are by weight.

*Example 1: Sulfuric Acid System*

5 ml of 96% sulfuric acid and 10 g (0.5 mol) of hydrogen fluoride were added to 3.0 g (0.1 mol) of paraformaldehyde. This reaction mixture was heated to 65°C. Thereafter, 13.4 g (0.08 mol) of 1,1,1,3,3,3-hexafluoroisopropyl alcohol was added drop-by-drop over 1 h. The reaction was conducted in the device described above and shown in Fig. 1. During this period, vapors were generated during the dropwise addition of the alcohol reactant. These vapors were collected in a cooled collector of a distillation set over a period of 2 h, using the nitrogen sweep technique and apparatus shown in Fig. 1. Thereafter, the material obtained in the cooled collector at the end of the 2 h was quenched on ice, neutralized with ammonia, and distilled.

The material from the cooled collector gave two fractions on distillation. Fraction 1, distilling between 25 and 58°C, provided 6.7 g of material containing 90% fluoromethyl-1,1,1,3,3,3-hexafluoroisopropyl ether, 3% of the initial alcohol reaction material, and 7% of a formal byproduct.

Fraction 2 from the cooled collector, distilling between 58 and 95°C, yielded 5.5 g of material containing 11% fluoromethyl-1,1,1,3,3,3-hexafluoroisopropyl ether, 42% of the alcohol starting material, 33% of a formal byproduct, and 13% of an acetal byproduct.

*Example 2: Phosphoric Acid System*

Paraformaldehyde (13 g), phosphoric acid (103 g), hydrogen fluoride (75 g) and hexafluoroisopropanol (47 g) were combined in the Fig. 1 reactor and heated at 65°C for 3 h. The distilled vapors were collected and neutralized with excess ammonia. The collected product (23 g) contained 88% hexafluoroisopropyl alcohol, 4.5% bis-hexafluoroisopropyl dioxymethylene acetal, 5% methyl hexafluoroisopropyl ether and 0.05% fluoromethyl-1,1,1,3,3,3-hexafluoroisopropyl ether. Phosphoric acid is a Brönsted acid having an acid equivalent number of −1.6.

*Example 3: Trifluoromethanesulfonic Acid System*

Paraformaldehyde (13 g), trifluoromethanesulfonic acid (39 g), hydrogen fluoride (43 g) and hexafluoroisopropyl alcohol (44 g) were combined in the Fig. 1 reactor and heated at 65°C for 3 h. The distilled vapors were collected and neutralized with excess ammonia. The isolated product (30 g) contained 66% fluoromethyl-1,1,1,3,3,3-hexafluoroisopropyl ether and 26% hexafluoroisopropyl alcohol.

*Example 4: Sulfuric Acid System*

Paraformaldehyde (13 g), concentrated sulfuric acid (136 g), hydrogen fluoride (53 g) and hexafluoroisopropyl alcohol (44 g) were combined in the Fig. 1 reactor and heated at 65°C for 3 h. The distilled vapors were collected and neutralized with excess ammonia. The isolated product (48 g) contained 84% fluoromethyl-1,1,1,3,3,3-hexafluoroisopropyl ether.

*Example 5: Fluorosulfonic/Sulfuric Acid System*

Paraformaldehyde (13 g), concentrated sulfuric acid (50 g), 20% sulfur trioxide in sulfuric acid (100 g), hydrogen fluoride (58 g) and hexafluoroisopropyl alcohol (45 g) were combined in the Fig. 1 reactor and heated at 65°C for 3 h. The distilled vapors were collected and neutralized with excess ammonia. The isolated product (45.5 g) contained 90% fluoromethyl-1,1,1,3,3,3-hexafluoroisopropyl ether.

## Claims

1. A method of synthesizing fluoromethylhexafluoroisopropyl ether in which formaldehyde, hydrogen fluoride and hexafluoroisopropyl alcohol are reacted together, characterized by the inclusion in the reaction mixture of dehydrating and fluoride ion generating agent which is either a Lewis acid or a protonating agent.

2. The method of claim 1, wherein the Lewis acid is titanium tetrachloride, aluminium trichloride, aluminium trifluoride or antimony pentafluoride.

3. The method of claim 1, wherein the agent is at least one Brönsted acid having a relative acid strength in excess of about 15.

4. The method of claim 3, wherein the Brönsted acid is sulfuric acid, fluorosulfonic acid or trifluoromethanesulfonic acid.

5. The method of any preceding claim, in which the mixture is maintained at a temperature of at least 50°C.

6. The method of any preceding claim, in which at least a 10% molar excess of formaldehyde is present, based on the hexafluoroisopropyl alcohol added.

7. The method of any preceding claim, in which at least a 400% molar excess of hydrogen fluoride is present, based on the hexafluoroisopropyl alcohol added.

8. The method of any preceding claim, wherein the recovered ether is further purified by distillation and the residue recycled to the reaction mixture.

9. The method of any preceding claim, wherein the formaldehyde is paraformaldehyde.

10. The method of synthesizing fluoromethyl-1,1,1,3,3,3-hexafluoroisopropyl ether which comprises mixing at a temperature of from 60 to 70°C 1,1,1,3,3,3-hexafluoroisopropyl alcohol with sulfuric acid and a stoichiometric excess of paraformaldehyde and hydrogen fluoride, condensing vapor generated by the mixture, and recovering the fluoromethyl-1,1,1,3,3,3-hexafluoroisopropyl ether from the condensed vapor.

## Patentansprüche

1. Verfahren zur Synthese von Fluoromethylhexafluoroisopropyläther, wobei Formaldehyd, Fluorwasserstoff und Hexafluorisopropylalkohol zusammen umgesetzt werden, gekennzeichnet durch die Verwendung in der Reaktionsmischung eines Dehydratisierungs- und Fluoridionenbildenden Mittels, welches entweder eine Lewis-Säure oder ein Protonierungsmittel ist.

2. Verfahren nach Anspruch 1, worin die Lewis-Säure Titantetrachlorid, Aluminiumtrichlorid, Aluminiumtrifluorid oder Antimonpentafluorid ist.

3. Verfahren nach Anspruch 1, worin das Mittel wenigstens eine Brönsted-Säure mit einer relativen Säurestärke von über etwa 15 ist.

4. Verfahren nach Anspruch 3, worin die Brönsted-Säure Schwefelsäure, Fluorsulfonsäure oder Trifluormethanschwefelsäure ist.

5. Verfahren nach einem der vorausgehenden Ansprüche, worin die Mischung bei einer Temperatur von wenigstens 50°C gehalten wird.

6. Verfahren nach einem der vorausgehenden Ansprüche, worin mindestens ein 10% molarer Überschuss an Formaldehyd vorhanden ist, bezogen auf den zugegebenen Hexafluorisopropylalkohol.

7. Verfahren nach einem der vorausgehenden Ansprüche, worin wenigstens ein 400% molarer Überschuss an Fluorwasserstoff vorhanden ist, bezogen auf den zugegebenen Hexafluorisopropylalkohol.

8. Verfahren nach einem der vorausgehenden Ansprüche, worin der gewonnene Äther durch Destillation weiter gereinigt und der Rückstand in die Reaktionsmischung zurückgeführt wird.

9. Verfahren nach einem der vorausgehenden Ansprüche, worin der Formaldehyd Paraformaldehyd ist.

10. Verfahren zur Synthese von Fluormethyl-1,1,1,3,3,3-hexafluorisopropyläther, welches umfasst: Das Mischen bei einer Temperatur zwischen 60 und 70°C von 1,1,1,3,3,3-Hexafluorisopropylalkohol mit Schwefelsäure und einem stöchiometrischem Überschuss an Paraformaldehyd und Fluorwasserstoff, das Kondensieren des durch das Mischen erzeugten Dampfes, und die Gewinnung von Fluormethyl-1,1,1,3,3,3,-hexafluorisopropyläther aus dem kondensierten Dampf.

## Revendications

1. Procédé de synthèse de l'éther fluorométhyl-hexafluoro-isopropylique selon lequel on fait réagir le formaldéhyde, l'acide fluorhydrique et l'alcool hexafluoro-isopropylique, caractérisé en ce qu'on inclut dans le mélange réactionnel un agent déshydratant et générateur d'ions fluorures, qui est soit un acide de Lewis, soit un agent donneur de protons.

2. Procédé selon la revendication 1, caractérisé en ce que l'acide de Lewis est le tétrachlorure de titane, le trichlorure d'aluminium, le trifluorure d'aluminium ou le pentafluorure d'antimoine.

3. Procédé selon la revendication 1, caractérisé en ce que l'agent est au moins un acide de Brönsted ayant une force relative d'acidité supérieure à environ 15.

4. Procédé selon la revendication 3, caractérisé en ce que l'acide de Brönsted est l'acide sulfurique, l'acide fluorosulfonique ou l'acide trifluoro-méthanesulfonique.

5. Procédé selon l'une des revendications 1 à 4, caractérisé en ce qu'on maintient le mélange à une température d'au moins 50°C.

6. Procédé selon l'une des revendications 1 à 5, caractérisé en ce qu'un excès molaire de formaldéhyde d'au moins 10% est présent, par rapport à l'alcool hexafluoro-isopropylique ajouté.

7. Procédé selon l'une des revendications 1 à 6, caractérisé en ce qu'un excès molaire d'acide fluorhydrique d'au moins 400% est présent, par rapport à l'alcool hexafluoro-isopropylique ajouté.

8. Procédé selon l'une des revendications 1 à 7, caractérisé en ce qu'on purifie encore l'éther récupéré par distillation et en ce qu'on recycle le résidu dans le mélange réactionnel.

9. Procédé selon l'une des revendications 1 à 8, caractérisé en ce que le formaldéhyde est le paraformaldéhyde.

10. Procédé de synthèse de l'éther fluorométhyl-1,1,1,3,3,3-hexafluoro-isopropylique, caractérisé en ce qu'on mélange à une température de 60 à 70°C de l'alcool 1,1,1,3,3,3-hexafluoro-isopropylique avec l'acide sulfurique et un excès stœchiométrique de paraformaldéhyde et d'acide fluorhydrique, on condense les vapeurs produites par le mélange et on récupère l'éther fluorométhyl-1,1,1,3,3,3-hexafluoro-isopropylique à partir des vapeurs condensées.

# FIG. I